# EUROPÄISCHE PATENTSCHRIFT

(11) **EP 3 579 817 B1**
(45) Veröffentlichungstag und Bekanntmachung des Hinweises auf die Patenterteilung: **27.09.2023**
(21) Anmeldenummer: 18700472.6
(22) Anmeldetag: 05.01.2018
(51) Int. Cl.: A61K 8/35, A61K 8/37, A61Q 17/04, A61K 8/04, A61K 8/06

(54) **SENSORISCH ATTRAKTIVE HYDRODISPERSION MIT UV-SCHUTZFILTERN 2-ETHYLHEXYLSALICYLAT UND 4-TERT-BUTYL-4'-METHOXY DIBENZOYLMETHAN**
SENSORILY ATTRACTIVE HYDRODISPERSION WITH THE UV PROTECTION FILTERS 2-ETHYLHEXYL SALICYLATE AND 4-TERT-BUTYL-4'-METHOXY DIBENZOYLMETHANE
HYDRODISPERSION À ATTRACTION SENSORIELLE PRÉSENTANT LES FILTRES DE PROTECTION CONTRE LES UV SALICYLATE DE 2-ÉTHYLHEXYLE ET 4-TERT-BUTYL-4'-MÉTHOXYDIBENZOYLMÉTHANE

(30) Priorität: 08.02.2017 DE 102017201948
(43) Veröffentlichungstag der Anmeldung: 18.12.2019
(73) Patentinhaber: Beiersdorf AG, 20253 Hamburg (DE)
(72) Erfinder: FRANCK, Kerstin, 25451 Quickborn (DE); RATSCHOW, Cecile, 37138 León (MX); MEYER, Christiane, 20357 Hamburg (DE)
(74) Vertreter: Beiersdorf AG
(86) Internationale Anmeldenummer: PCT/EP2018/050266
(87) Internationale Veröffentlichungsnummer: WO 2018/145831

(56) Entgegenhaltungen:
- EP-A1- 3 173 129
- WO-A2-2004/091639
- DE-A1-102013 217 247
- US-A1- 2011 165 092
- US-A1- 2016 008 244

## Beschreibung

Die Erfindung umfasst Hydrodispersion mit Verdickungsmitteln, Wachsen, Ölen und UV-Schutzfiltern.

Unter Kosmetik kann man alle Maßnahmen zusammenfassen, die aus ästhetischen Gründen Veränderungen an Haut und Haaren vornehmen oder zur Körperreinigung angewendet werden. Kosmetik bedeutet also, das Körperäußere zu pflegen, zu verbessern und zu verschönern, um auf sichtbare, fühlbare und riechbare Weise sowohl den Mitmenschen als auch sich selbst zu gefallen. Schon vor Jahrtausenden wurde Kosmetik vom Menschen zu diesem Zweck angewandt. Man färbte Lippen und Gesicht, salbte sich mit wertvollen Ölen und badete in duftendem Wasser.

Häufige Erscheinungsformen kosmetischer oder dermatologischer Zubereitungen sind feindisperse Mehrphasensysteme, in welchen eine oder mehrere Fett- bzw. Ölphasen neben einer bzw. mehreren Wasserphasen vorliegen. Von diesen Systemen sind wiederum die eigentlichen Emulsionen die am weitesten verbreiteten.

Aber auch emulgatorarme bzw. -freie Zubereitungen auf Basis sogenannter Hydrodispersionen sind bekannt. Hydrodispersionen stellen Dispersionen einer flüssigen, halbfesten oder festen inneren (diskontinuierlichen) Lipidphase in einer äußeren wässrigen (kontinuierlichen) Phase dar. Hydrodispersionen sind - wie auch Emulsionen, die sich durch eine ähnliche Phasenanordnung auszeichnen - metastabile Systeme und daher geneigt, in einen Zustand zweier in sich zusammenhängender diskreter Phasen überzugehen. In einer klassischen O/W-Emulsion verhindert die Wahl eines geeigneten Emulgators die Phasentrennung. Im Gegensatz zur klassischen Emulsionen enthalten Hydrodispersionen aber nur sehr geringe Mengen an Emulgatoren (bis zu 2 Gew.-%) bzw. können sogar gänzlich frei von Emulgatoren sein.

Bei Hydrodispersionen, welche aus einer flüssigen Lipidphase in einer äußeren wässrigen Phase bestehen, wird die Stabilität des Mehrphasensystems üblicherweise dadurch gewährleistet, dass in der wässrigen Phase mit Hilfe eines Gelbildners bzw. Verdickungsmittels ein Gelgerüst aufgebaut wird, in welchem die Lipidtröpfchen stabil suspendiert sind.

Als Verdickungsmittel sind Homopolymere oder Copolymere von Acrylsäure und/oder Acrylamid und deren Derivaten bekannt. Formulierungen auf Polyacrylatbasis zeigen jedoch häufig keine nachhaltigen Pflegeeffekte. Gel-Formulierungen sind außerdem salzempfindlich, so dass sie beim Auftragen auf die Haut durch die Präsenz von Salzen häufig brechen.

Nachteil der Hydrodispersionen des Standes der Technik ist ferner, dass diese bei einem höheren Gehalt an Lipiden (größer 4 Gew.-%) zur Phasentrennung neigen, die insbesondere bei höheren Temperaturen (≥ 40 °C) auftreten kann.

Des Weiteren fühlen sich Hydrodispersionen im Vergleich zu Emulsionen häufig klebrig an. Ein weiterer Nachteil von Hydrodispersionen, insbesondere solcher, die Acrylate als Gelbildner enthalten, ist der unvorteilhafte Abrieb, wenn die auf die Haut aufgetragene Hydrogelschicht leicht und in unschöner Weise ("Röllchenbildung") abgetragen wird.

Eine für den Verbraucher sehr wesentliche, dabei aber nur schwer quantitativ messbare Eigenschaft kosmetischer Produkte ist ihre Textur. Unter dem Begriff "Textur" werden diejenigen Eigenschaften eines Kosmetikums verstanden, die auf den Gefügebau der Zubereitung zurückgehen, durch Tast- und Berührungssinne empfunden und ggf. in mechanischen oder rheologischen Fließeigenschaften ausgedrückt werden können. Die Textur kann insbesondere mittels Sensorik getestet werden. Die gegebenenfalls mit Hilfe von Zusatzstoffen beeinflussbare Textur kosmetischer Produkte ist für den Verbraucher von nahezu gleicher Bedeutung wie deren objektiv feststellbaren Wirkungen.

Mit dem Begriff "Sensorik" wird die wissenschaftliche Disziplin bezeichnet, die sich mit der Bewertung von kosmetischen Zubereitungen auf Grund von Sinneseindrücken befasst. Die sensorische Beurteilung eines Kosmetikums erfolgt anhand der visuellen, olfaktorischen und haptischen Eindrücke.
- *Visuelle Eindrücke:* alle mit dem Auge wahrnehmbaren Merkmale (Farbe, Form, Struktur).
- *Olfaktorische Eindrücke:* alle beim Einziehen von Luft durch die Nase wahrnehmbaren Geruchseindrücke, die häufig in Anfangsgeruch (Kopfnote), Hauptgeruch (Mittelnote, Körper) und Nachgeruch (Ausklang) differenziert werden können. Auch die erst bei der Anwendung freigesetzten flüchtigen Stoffe tragen zum olfaktorischen Eindruck bei.
- *Haptische Eindrücke:* alle Empfindungen des Tastsinns, die vornehmlich Gefüge und Konsistenz des Produktes betreffen.

Die sensorische Analyse macht von der Möglichkeit Gebrauch, den sensorischen Gesamteindruck eines Produktes integral zu erfassen. Nachteile der sensorische Analyse sind die Subjektivität des Eindrucks, eine leichte Beeinflussbarkeit der Prüfpersonen und die dadurch bedingte starke Streuung der Ergebnisse. Diesen Schwächen begegnet man heute durch den Einsatz von Gruppen geschulter Prüfpersonen, gegenseitige Abschirmung der Prüfer sowie statistische Auswertung der meist zahlreichen Analysendaten.

In der WO 2005097057 A1 werden Gelformulierungen offenbart, die polymere Gelbbildnern, Wasser, Öle sowie mindestens ein Wachs, das einen Schmelzpunkt von 30°C und mehr besitzt, umfassen. Die Formulierungen sind frei von kationischen oder anionischen Emulgatoren oder Tensiden.

In der WO 2005097055 A1 werden Gelformulierungen beschrieben, die polymere Gelbildner, Wasser, eine bei 25°C flüssige Ölphase und mindestens eine Wachskomponente mit einem Schmelzpunkt von wenigstens 30°C ausgewählt aus der Gruppe der Pentaerythritester, der Dipentaerythritester und/oder der Tripentaerythritester, umfassen.

Die sensorischen Eigenschaften dieser Gelformulierungen stellen sich jedoch als nicht so attraktiv dar. Der Rückstand fühlte sich sehr wachsig an.

Wünschenswert ist es daher Hydrodispersionen bereit zu stellen, die sensorisch verbesserte Eigenschaften aufweisen und insbesondere deren Rückstand weniger wachsig erscheint.

In der Patentanmeldung DE 102012221224 werden Hydrodispersion beschrieben, die Wasser, ein oder mehrere Verdickungsmittel, gegebenenfalls ein oder mehrere Wachse und gegebenenfalls ein oder mehrerer Öle, sowie Ethylhexylglycerin und/oder Caprylylglycol enthalten.

In der Patentanmeldung DE 102012221227 werden Hydrodispersion beschrieben, die Verdickungsmittel, Wachse und Öle enthalten. Die Wachse und die Gesamtzubereitung weisen spezifische Schmelzbereiche auf, die für ein angenehmes, sensorisch leichtes Profil und gute Pflegeeffekte sorgen. Die Hydrodispersionen enthalten in der Fettphase kaum flüssige Öle sondern zum größten Teil Wachse mit einem sehr definierten Schmelzbereich. Dieser Schmelzbereich ist so gewählt, dass die Wachse bei Raumtemperatur fest sind und somit in der Lage dem Produkt eine hohe Konsistenz zu verleihen, während sie auf der Haut beim Verreiben schmelzen und dadurch einen kühlenden Effekt besitzen. Diese spezielle Sensorik wird nach Ansicht der Experten durch die Zugabe flüssiger Öle immer weiter gestört, bis der Effekt schließlich ganz verschwindet. Theoretisch sollte daher der Zusatz von ölbasierten Lichtfiltersubstanzen zu einer deutlichen Veränderung der Sensorik der Hydrodispersion führen und der Zusatz von wasserlöslichen Filtern (wie z.B. Phenylbenzimidazole Sulfonic Acid) sollte die Sensorik deutlich weniger negativ beeinflussen.

In den Patentanmeldungen DE 102012221227 und DE 102012221224 wird der generelle Einsatz von Lichtfiltersubstanzen wird erwähnt. Überraschenderweise wurde aber nun gefunden, dass nur öllösliche Filtersubstanzen geeignet ist, um die sensorischen Vorteile der beschriebenen Hydrodispersionen weiterhin für den Verbraucher erlebbar zu machen.

Insbesondere eine Kombination aus Ethylhexyl Salicylate und Butyl Methoxydibenzoylmethane führte zu besonders guten Ergebnissen in der Sensorik. US 2016/008244 A1 (CASTRO MAURICIO [US]) (2016-01-14) beschreibt Hydrodispersionen mit den UV-Schutzfiltern 2-Ethylhexylsalicylat und 4-tert-Butyl-4'-methoxydibenzoylmethan.

Aufgabe war es kosmetische oder dermatologische Hydrodispersionen bereit zu stellen, die mit Wachsen formuliert, ein angenehmes, sensorisch leichtes Profil und gute Pflegeeffekte aufweisen und bei Applikation auf der Haut nicht brechen. Diese Hydrodispersionen sollten UV-Schutz bieten. Die resultierenden Formeln mit UV-Schutz sollten sensorisch möglichst ähnlich sein zu den Hydrodispersionen ohne Lichtschutzfilter.

Erfindungsgemäß werden dies Aufgaben gelöst durch kosmetische oder dermatologische Zubereitungen in Form von Hydrodispersionen, gemäss Anspruch 1
a.) welche aus einer flüssigen Lipidphase in einer äußeren wässrigen Phase bestehen,
b.) wobei in der wässrigen Phase mit Hilfe eines oder mehrerer Gelbildners und/oder eines oder mehrerer Verdickungsmittel ein Gelgerüst aufgebaut wird, in welchem die Lipidtröpfchen stabil suspendiert sind,
c.) wobei die Ölphase ein oder mehrere Wachse enthält,
d.) wobei die Ölphase einen Gehalt an 2-Ethylhexylsalicylat und 4-tert-Butyl-4'-methoxydi-benzoylmethan aufweist und
e.) die Zubereitungen höchstens 2 Gew.-% an einem oder mehreren Emulgatoren enthält, bezogen auf das Gesamtgewicht der Zubereitungen, und bevorzugt gänzlich frei von Emulgatoren ist.

Bevorzugt werden die Konzentrationen an 2-Ethylhexylsalicylat und 4-tert-Butyl-4'-methoxy-dibenzoylmethan aus folgenden Bereichen gewählt:
2-Ethylhexylsalicylat 0,5 - 20 Gew.-%, bevorzugt 1 - 10 Gew.-%, insbesondere bevorzugt 3 - 5 Gew.-%, jeweils bezogen auf das Gesamtgewicht der Zubereitungen.
4-tert-Butyl-4'-methoxydibenzoylmethan 0,1 - 10 Gew.-%, bevorzugt 0,2 - 2 Gew.-%, insbesondere bevorzugt 0,5 - 1 Gew.-%, jeweils bezogen auf das Gesamtgewicht der Zubereitungen.

Weiterhin ist bevorzugt, Gewichtsverhältnisse von 2-Ethylhexylsalicylat zu 4-tert-Butyl-4'-methoxydibenzoylmethan aus dem Bereich von 20 : 1 bis 1 : 5 zu wählen, bevorzugt von 15 : 1 bis 1 : 1, ganz besonders bevorzugt von 10 : 1 bis 5 : 1.

Vorteilhaft ist ferner, wenn das Wachs oder die Wachse so ausgewählt wird oder werden, wenn mindestens eine Wachskomponente einen Schmelzpunkt Tₒₙₛₑₜ kleiner 30°C hat und die gesamte Formulierung vorteilhaft mindestens einen Schmelzbereich mittels DSC zwischen 5°C und 30°C aufweist

Die Dispersion umfasst vorteilhaft mindestens einen Schmelzbereich zwischen 5°C und 30°C nach DSC. Ein weiterer oder mehrere Schmelzbereiche können ausserhalb dieser Temperturspanne liegen.

Entgegen den Gelformulierungen des Standes der Technik sind die erfindungsgemäßen Hydrodispersionen sensorisch attraktiv und pflegend.

Die erfindungsgemäße Dispersion weist eine überraschend reichhaltige Textur bei der Entnahme auf.

Aufgrund der erfindungsgemäßen Wachsschmelzkaskade zeigen die Zubereitungen zudem einen Kühleffekt beim Verteilen und eine sehr leichte Verteilbarkeit auf der Haut.

Das oder die Verdickungsmittel werden erfindungsgemäß aus einer oder mehreren der folgenden Gruppen gewählt:
- organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein, Xanthan Gum
- organische, abgewandelte Naturstoffe, wie z. B. Carboxymethylcellulose und andere Celluloseether, Hydroxyethyl- und -propylcellulose und mikrokristalline Cellulose oder dergleichen, .

Ferner können optional folgende Verdickungsmittel hinzugefügt werden:
° Homopolymere oder Copolymere von Acrylsäure und/oder Acrylamid und deren Derivaten, oder
   - organische, vollsynthetische Verbindungen, wie z. B. Polyacryl- und Polymethacryl-Verbindungen, Vinylpolymere, Polycarbonsäuren, Polyether, Polyimine, Polyamide, Polyurethane, AMPS Copolymere wie z.B. Ammonium Acryloyldimethyltaurate/VP Copolymer und Natrium Acrylate/ Acryloyldimethyltaurate/Dimethylacrylamide Crosspolymer, Acrylic Acid/VP Crosspolymer, PVP, Acrylamide/AmmoniumAcrylate Copolymer.

Besonders bevorzugte, optionale Verdickungsmittel im Sinne der vorliegenden Erfindung sind Polyacrylate, d. h. Polymere auf Basis von Estern der Acrylsäure (Polyacrylsäureester) der allgemeinen Strukturformel 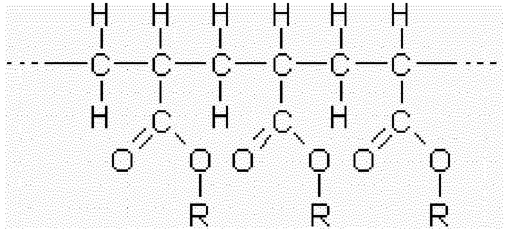 worin R für lineare, verzweigte oder cyclische, ggf. funktionelle Substituenten (z. B. Hydroxy-, Amin- oder Epoxid-Gruppen) enthaltende Alkyl-Reste steht, z. B. für Methyl-, Ethyl-, Isopropyl-, tert-Butyl-, Cyclohexyl-, 2-Ethylhexyl-, Dodecyl-, 2-Hydroxyethyl- und 2-Dimethylaminoethyl-.

Ganz besonders bevorzugt ist die Verwendung eines Gemisches aus vorgenannten Verdickungsmitteln. Dabei ist der Einsatz folgender Hydrocolloide besonders bevorzugt:
1) Polysaccharide mit der INCI-Bezeichnung Xanthan Gum, beispielsweise das unter der Handelsbezeichnung Keltrol CG-RD bei der Fa. CP Kelco erhältliche.
2) Polyacrylate mit der INCI-Bezeichnung Acrylates/C10-30 Alkyl Acrylate Crosspolymer, beispielsweise das unter der Handelsbezeichnung Pemulen TR1 bei der Fa. Lubrizol Advanced Materials erhältliche.
3) Polyacrylate mit der INCI-Bezeichnung Sodium Polyacrylate, beispielsweise das unter der Handelsbezeichnung Cosmedia SP bei der Fa. BASF Personal Care and Nutrition erhältliche.

Der Anteil an Verdickungsmitteln wird bevorzugt im Bereich von 0,1 bis 2 Gew.-% gewählt, bezogen auf die Gesamtmasse der Zubereitung.

Unter dem Begriff Wachs werden üblicherweise alle natürlichen oder künstlich gewonnenen Stoffe und Stoffgemische mit folgenden Eigenschaften verstanden:
Die erfindungsgemäßen Wachse sind bei Raumtemperatur (20°C) von fester bis brüchig harter Konsistenz, grob bis feinkristallin, durchscheinend bis trüb und schmelzen oberhalb von 20°C ohne Zersetzung. Sie sind schon wenig oberhalb des Schmelzpunktes niedrigviskos und nicht fadenziehend und zeigen eine starke temperaturabhängige Konsistenz und Löslichkeit. Erfindungsgemäß einsetzbar ist eine Wachskomponente oder ein Gemisch von Wachskomponenten, die bei einer Temperatur Tₒₙₛₑₜ<30°C anfängt zu schmelzen aber bei RT (20°C) noch fest ist/sind. D.h. mindestens ein Wachs weist einen Schmelzpunkt Tₒₙₛₑₜ < 30°C auf oder vorteilhaft alle gewählten Wachse weisen diesen Schmelzpunkt auf.

Tₒₙₛₑₜ wird mittels DSC bestimmt.

DSC (Differential Scanning Calorimetry) ist ein thermisches Verfahren zur Messung von abgegebener/aufgenommener Wärmemenge einer Probe bei isothermer Arbeitsweise, Aufheizung oder Abkühlung (siehe DIN 53765, DIN 51007, ASTM E 474, ASTM D 3418). DSC ist eine vergleichende Messmethode, die die Bestimmung von Wärmemengen physikalischer und chemischer Prozesse ermöglicht. Wenn ein Material seinen physikalischen Zustand ändert, wie z.B. Schmelzen oder Umwandlung einer Kristallform in eine andere oder wenn es chemisch reagiert, wird Wärme dabei aufgenommen oder abgegeben. Diese Wärmemengen sind mit Hilfe der DSC quantitativ messbar.

Die Methode verläuft zyklisch, so dass nach der ersten Aufheizkurve ein definiertes Abkühlen stattfindet und anschließend die Probe noch einmal im angegebenen Temperaturbereich aufgeheizt wird. Man erhält somit zweierlei Informationen: In der ersten Aufheizkurve sind alle thermische Effekte inklusiv Vorgeschichte erkennbar. In der zweiten Aufheizkurve ist die Vorgeschichte eliminiert worden und das reine thermische Verhalten der Probe unter definierten Abkühlbedingungen ist auswertbar. Die Schmelztemperatur Tₒₙₛₑₜ der Wachse wird bei der zweiten Aufheizkurve ermittelt. Dagegen ist der Schmelzbereich der Hydrodispersion zwischen 5°C und 30°C nach DSC der in der ersten Aufheizkurve ermittelte Bereich.

Als Wachse können erfindungsgemäß auch Fette und fettähnlichen Substanzen mit wachsartiger Konsistenz eingesetzt werden, solange sie einem Tₒₙₛₑₜ < von 30°C haben. Hierzu gehören u.a. Fette (Triglyceride), Mono und Diglyceride, natürliche und synthetische Wachse, Fett-und Wachsalkohole, Ester von Fettalkoholen und Fettsäuren sowie Fettsäureamide oder beliebige Gemische dieser Substanz.

Besonders bevorzugt werden die Wachse aus der Gruppe der Fette, insbesondere aus:
- Shorea Stenoptera Seed Butter (Tₒₙₛₑₜ=14,9°C)
- Hydrogenated Vegetable Oil (Tₒₙₛₑₜ=18,9°C)
- Hydrogenated Coco-Glycerides (Tₒₙₛₑₜ=26,8°C)
- Butyrospermum Parkii Butter (Tₒₙₛₑₜ=28,3°C)
- Theobroma Cacao (Cocoa) Seed Butter (Tₒₙₛₑₜ=14,5°C)
- Mango Butter (Tₒₙₛₑₜ=19,4°C)
- Hydrogenated Palm Kernel Glycerides and Hydrogenated Palm Glycerides (Lipocire A, Gattefossé) (Tₒₙₛₑₜ=20,3°C)
- Acacia Decurrens/Jojoba/Sunflower Seed Wax Polyglyceryl-3 Esters (Tₒₙₛₑₜ=14,4°C)
- aus der Gruppe der Ester aus Fettsäuren, insbesondere aus
- Cetearyl Nonanoate (Tₒₙₛₑₜ=24°C)
- Methyl Palmitate (Tₒₙₛₑₜ=27,7°C),
- sowie aus der Gruppe der Fettalkohole, insbesondere aus
- Cetearyl Alcohol (Tₒₙₛₑₜ=28, 1°C)
- gewählt.

Die erfindungsgemäßen Hydrodispersion kann neben den Wachsen auch andere Öle oder Lipide enthalten, vorteilhaft zu einem Anteil bis zu 10 Gew.%, bezogen auf die Gesamtmasse der Zubereitung. Dabei ist jedoch darauf zu achten, dass die Gesamtzubereitung mindestens einen Schmelzbereich im erfindungsgemäßen Bereich von 5°C bis 30°C aufweisen sollte.

Dies kann man erreichen, indem man erfindungsgemäße Wachse wählt, die einen Tₒₙₛₑₜ<30°C haben, und indem man die erhaltene Dispersionen entsprechend testet.

Vorteilhaft ist, dass die Gesamtmenge der Wachse größer ist als die Gesamtmenge der Öle.

Zusätzliche Lipide bzw. Öle werden bevorzugt gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnussöl, Rapsöl, Arganöl, Mandelöl, Palmöl, Kokosöl, Rizinusöl, Weizenkeimöl, Traubenkernöl, Distelöl, Nachtkerzenöl, Macadamianußöl und dergleichen mehr.

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodecanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Die erfindungsgemäßen Zubereitungen können optional einen Gehalt an cyclischen und/oder linearen Silikonölen aufweisen.

Vorteilhaft werden Cyclomethicone, insbesondere Octamethylcyclotetrasiloxan, Cyclomethicone D5 und/oder Cyclomethicone D6 als erfindungsgemäßes Silikonöl eingesetzt. Ein weiteres erfindungsgemäß vorteilhaftes Silikonöl ist Dimethicon (auch: Dimethylpolysiloxan bzw. Polydimethylsiloxan mit der INCI-Bezeichnung Dimethicone).

Aber auch andere Silikonöle sind vorteilhaft im Sinne der vorliegenden Erfindung zu verwenden, beispielsweise Hexamethylcyclotrisiloxan, Poly(methylphenylsiloxan), Phenyltrimethicon, Phenyldimethicon, Hexamethylcyclotrisiloxan, Polydimethylsiloxan, Poly(methylphenyl-siloxan), Cetyldimethicon, Behenoxydimethicon.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Poly-siloxan-polyalkyl-polyethercopolymere wie das Cetyl-Dimethicon-Copolyol oder auch das Lauryl-Methicon-Copolyol.

Der Anteil an ein oder mehreren Silikonöle, besonders bevorzugt Dimethicon, wird bevorzugt gewählt im Bereich von 0,1 bis 10 Gew.-%, besonders 0,5 bis 3 Gew.-%, jeweils bezogen auf das Gesamtgewicht der Zubereitung.

In einer besonders bevorzugten Ausführungsform enthalten die Zubereitungen im Sinne der vorliegenden Erfindung sogenannte Moisturizer. Als Moisturizer werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure, Harnstoff und Glyceryl Glucoside.

Vorteilhaft wird die Menge an Moisturizern, eine oder mehrere Verbindungen, aus dem Bereich von 1 bis 25 Gew.-%, bevorzugt von 3 bis 20 Gew.-%, besonders bevorzugt von 5 bis 15 Gew.-% - jeweils bezogen auf das Gesamtgewicht der Zubereitung - gewählt.

Vorteilhaft umfasst die erfindungsgemäße Zubereitung Glycerin, insbesondere zu einem Ateil von 1% bis 12 Gew.%, bezogen auf die Gesamtmasse der Zubereitung.

Die kosmetische Zusammensetzungen im Sinne der vorliegenden Erfindung können, je nach ihrem Aufbau, beispielsweise verwendet werden als Hautschutzcreme, Reinigungsmilch, Sonnenschutzlotion, Nährcreme, Tages- oder Nachtcreme usw.

Vorteilhaft wird die Hydrodsipersion oder Zubereitung als Hautpflegecreme verwendet.

Die Vorteile der erfindungsgemäßen Hydrodispersion sind deren leichte Verteilbarkeit, erfrischender Effekt bei der Applikation und dennoch eine pflegende Wirkung, die insbesondere durch die Wachse hervorgerufen werden.

Die erfindungsgemäße Hydrodisersion ist besonders hautverträglich, da wenig bzw. keine Öle, insbesondere keine spreitende Öle, wenig Emulgatoren und wenig Konservierungsmittel enthalten sind. Eine Anwendung der Hydrodispersion am Auge ist damit problemlos möglich.

Die Zusammensetzungen können auch weitere UV-Filter enthalten gewählt aus der Gruppe der Verbindungen 2-Phenylbenzimidazol-5-sulfonsäure und/oder deren Salze, Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)ben zolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)-campher; 3-Benzylidencampher; Terephthalidendicamphersulfonsäure; 2-Ethylhexyl-2-cyano-3,3-diphenylacrylat; 4-(Dimethylamino)-benzoesäure(2-ethylhexyl)ester; Diethylamino Hydroxybenzoyl Hexyl Benzoate, 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester;Homomenthylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer;Dioctylbutylamidotriazon (INCI: Diethylhexyl-Butamidotriazone); 2,4-bis-[5-1(dimethylpropyl) benzoxazol-2-yl-(4-phenyl)-imino]-6-(2-ethylhexyl)-imino-1,3,5-triazin mit der (CAS Nr.288254-16-0); 4,4',4"-(1,3,5-Triazin-2,4,6-triyltriimino)-tris-benzoësäure-tris(2-ethylhexylester)(auch: 2,4,6-Tris-[anilino-(p-carbo-2'-ethyl-1'-hexyloxy)]-1,3,5-triazin (INCI: Ethylhexyl Triazone);2,4-Bis-{[4-(2-ethyl-hexyloxy)-2-hydroxy]-phenyl}-6-(4-methoxyphenyl)-1,3,5-triazin; 2,4,6-Tribiphenyl-4-yl-1,3,5-triazin; Merocyanine, Butyl Methoxydibenzoylmethane, Octocrylene, sowie Titandioxide und Zinkoxide.

Die kosmetischen oder dermatologischen Zubereitungen gemäß der Erfindung können ferner kosmetische Hilfsstoffe und Wirkstoffe enthalten, wie sie üblicherweise in solchen Zubereitungen verwendet werden, z. B. Konservierungsmittel, Konservierungshelfer, Bakterizide, Substanzen zum Verhindern des Schäumens, Farbstoffe und Farbpigmente, Verdickungsmittel, anfeuchtende und/oder feuchthaltende Substanzen, Anti-Aging-Substanzen oder andere übliche Bestandteile einer kosmetischen oder dermatologischen Formulierung wie Polyole, Polymere, Schaumstabilisatoren, organische Lösungsmittel oder Silikonderivate, sofern der Zusatz die geforderten Eigenschaften hinsichtlich der Sensorik und Pflegeeffekte nicht beeinträchtigt. Folgende Wirkstoffe können z.B. enthalten sein, gewählt aus der Gruppe der Verbindungen Gylcyrrhetinsäure, Harnstoff, Arctiin, alpha-Liponsäure, Folsäure, Phytoen, D-Biotin, Coenzym Q10, alpha-Glucosylrutin, Tocopheryl Acetate, Carnitin,Carnosin, Coffein, natürliche und/oder synthetische Isoflavonoide, Glycerylglucose, Kreatin, Kreatin in, Taurin, Magnolia, β-Alanin und/oder Licochalcon A.

Die erfindungsgemäßen Hydrodispersionen stellen bevorzugt kosmerische und/oder dermatologische Zubereitungen dar.

Stabile kosmetische Zubereitungen sind solche, die über einen längeren Zeitraum sowie bei Temperaturwechsel keinerlei Phasentrennung, Viskositätsänderungen, Koaleszenz, Ostwald-Reifung und/oder Aufrahmungen zeigen.

Eine Phasentrennung der Zubereitung zwischen Lipdphase und Wasserphase, auch als Brechen bezeichnet, ist daher während der Lagerung und Anwendung nicht erwünscht.

Die erfindungsgemäßen Zubereitungen sind stabil und zeigen keinerlei Phasentrennung bei der Applikation auf die Haut.

Die erfindungsgemäßen Zubereitungen sind bevorzugt frei von anionischen und/oder kationischen Emulgatoren/Tenside.

Sind jedoch aufgrund von Rohstoffverunreinigungen oder sonstigen Einschleppungen anionische oder kationische emulgierende Stoffe oder waschaktive Substanzen in den Zubereitungen enthalten, so gelten diese Zubereitungen erfindungsgemäß immer noch als "frei von" Emulgatoren und Tensiden, sofern deren Anteil nicht über 0,1 Gew.-%, bezogen auf die Gesamtmasse der Zubereitung, beträgt.

Verdickungsmittel, die u.U. auch emulgierende Eigenschaften haben können, werden jedoch nicht zu den auszuschließenden Emulgatoren gezählt, da deren Hauptfunktion nicht die eines Emulgators ist.

### Sensorische Evaluierung erfindungsgemäßer Zusammensetzungen:

Die Testprodukte werden von trainierten Probanden (Panelisten, ca. 10-15) mittels einer hochstandardisierten Vorgehensweise bewertet.

Die Produkte werden dazu auf dem Unterarm appliziert, verteilt und direkt beurteilt. Zur Bewertung wird eine Skala von 0 (trifft nicht zu) bis 10 (sehr stark ausgeprägt) verwendet. Zur Auswertung wird der Mittelwert der Bewertungen aller Panelisten herangezogen.

Ergebnis (n=12)

| | Ohne Lichtfilter (Vergleich) | Mit öllöslichem Filter (erfindungsgemäß) |
|---|---|---|
| Verteilbarkeit (5 Kreise) | 6,6 | 6,5 |
| Konsistenz (5 Kreise) | 3,6 | 3,7 |
| Feuchtigkeit (5 Kreise) | 5,5 | 5,0 |
| Feuchtigkeit (20 Kreise) | 3,1 | 3,0 |
| Weißeln 4x | 1,5 | 2,4 |
| Weißeln 20x | 0,8 | 1,6 |
| Gleitvermögen | 6,5 | 6,5 |
| Ölig sofort | 2,0 | 2,0 |
| Ölig 5 min. | 0,8 | 0,8 |

| | Ohne Lichtfilter (Vergleich) | Mit wasserlöslichem Filter (nicht erfindungsgemäß) |
|---|---|---|
| Verteilbarkeit (5 Kreise) | 6,6 | 6,2 |
| Konsistenz (5 Kreise) | 3,6 | 4,0 |
| Feuchtigkeit (5 Kreise) | 5,5 | 4,0 |
| Feuchtigkeit (20 Kreise) | 3,1 | 2,0 |
| Weißeln 4x | 1,5 | 3,3 |
| Weißeln 20x | 0,8 | 2,3 |
| Gleitvermögen | 6,5 | 6,2 |
| Ölig sofort | 2,0 | 1,8 |
| Ölig 5 min. | 0,8 | 0,6 |

Nachfolgende Beispiele erläutern die erfindungsgemäßen Hydrodispersionen. Beispiele 3 und 6 sind nicht erfindungsgemäss. Die angeführten Zahlen sind, sofern nichts anderes angegeben, Gewichtsanteile bezogen auf die Gesamtmasse der Zubereitung.

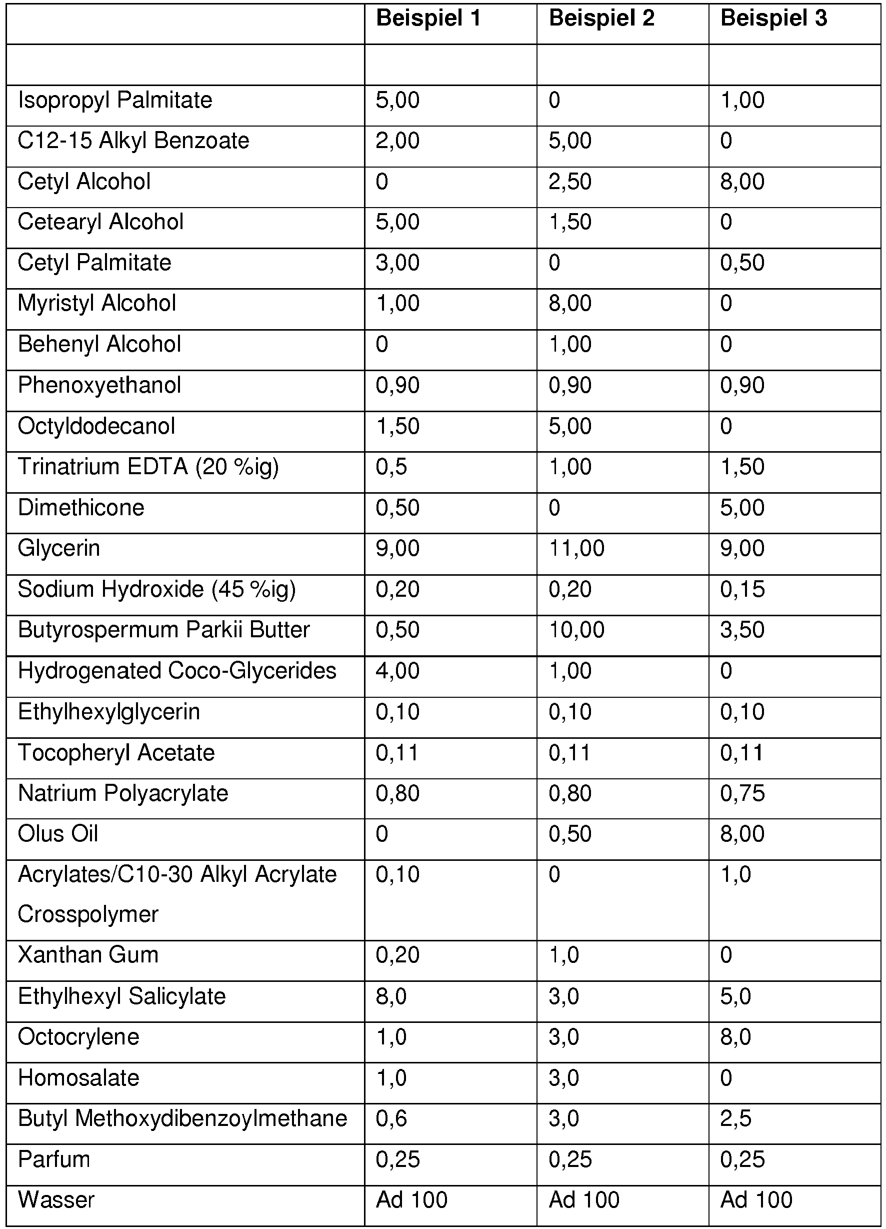

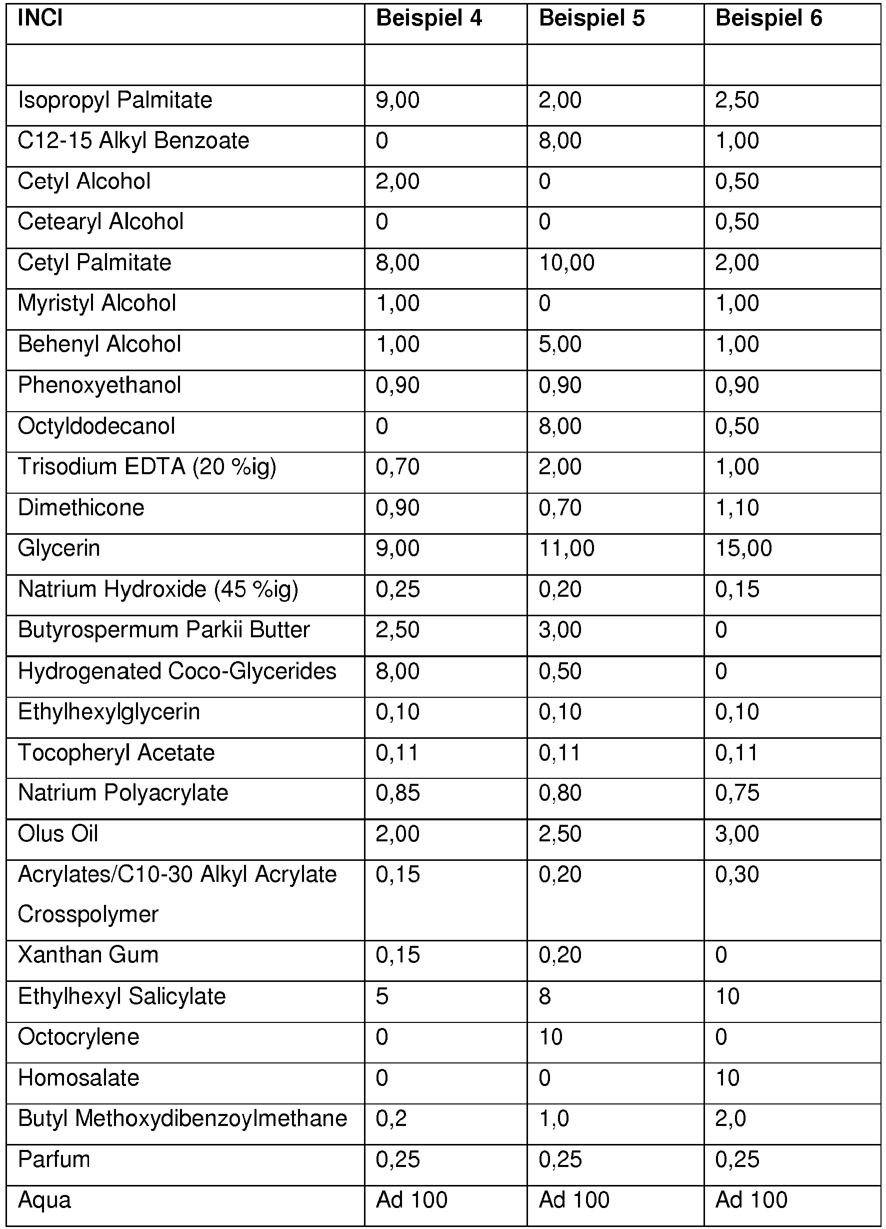

## Patentansprüche

1. Kosmetische oder dermatologische Zubereitungen in Form von Hydrodispersionen,
a) welche aus einer flüssigen Lipidphase in einer äußeren wässrigen Phase bestehen,
b) wobei in der wässrigen Phase mit Hilfe eines oder mehrerer Gelbildners und/oder eines oder mehrerer Verdickungsmittel ein Gelgerüst aufgebaut wird, in welchem die Lipidtröpfchen stabil suspendiert sind,
c) wobei die Ölphase ein oder mehrere Wachse enthält,
d) wobei die Ölphase einen Gehalt an 2-Ethylhexylsalicylat und 4-tert-Butyl-4'-methoxydibenzoylmethan aufweist und
e) die Zubereitungen frei von Emulgatoren ist,
**dadurch gekennzeichnet, dass** sie folgende Zusammensetzungen aufweisen:
f) 60 - 95 Gew.-%, insbesondere 70 - 85 Gew.-% an Wasser
g) 0,05 - 5 Gew.-% wenigstens eines Verdickungsmittels,
h) bis 20 Gew.-% eines oder mehrerer Öle, insbesondere mehr als 0,5 Gew.- %, vorteilhaft 1 bis 10 Gew.-%, jeweils bezogen auf die Gesamtmasse der Dispersion wobei die Ölphase 2 bis 20 Gew.-%, bezogen auf die Gesamtmasse der Dispersion, eines oder mehrerer Wachse, enthält, wobei mindestens ein Wachs einen Schmelzpunkt T_{onSet} < 30°C aufweist,
i) das oder die Verdickungsmittel ausgewählt werden aus der Gruppe
• der organischen, natürlichen Verbindungen, wie insbesondere Agar-Agar, Carrageen, Tragant, Gummi arabicum, Alginate, Pektine, Polyosen, Guar-Mehl, Johannisbrotbaumkernmehl, Stärke, Dextrine, Gelatine, Casein, Xanthan Gum,
• der organischen, abgewandelten Naturstoffe, wie insbesondere Carboxymethylcellulose, Hydroxyethyl- und/oder -propylcellulose, mikrokristalline Cellulose,

2. Zubereitungen nach einem der vorstehenden Ansprüche, **dadurch gekennzeichnet, dass** dass als zusätzliches Verdickungsmittel Natriumpolyacrylat gewählt wird

3. Zubereitungen nach einem der vorstehenden Ansprüche, **dadurch gekennzeichnet, dass** der Anteil an Verdickungsmitteln im Bereich 0,1 bis 2 Gew.-%, bezogen auf die Gesamtmasse der Hydrodispersionszubereitung, gewählt wird.

4. Zubereitungen nach einem der vorstehenden Ansprüche, **dadurch gekennzeichnet, dass** das Wachs oder die Wachse mit einem T_{onSet} unterhalb von 30°C gewählt werden aus der Gruppe der Fette (Triglyceride), Mono- und Diglyceride, natürliche und synthetische Wachse, Fett- und Wachsalkohole, Fettsäuren, Ester von Fettalkoholen und Fettsäuren sowie Fettsäureamide oder beliebige Gemische dieser Substanzen.

5. Zubereitungen nach einem der vorstehenden Ansprüche, **dadurch gekennzeichnet, dass** das Wachs oder die Wachse gewählt werden aus der Gruppe Shorea Stenoptera Seed Butter, Hydrogenated Vegetable Oil, Hydrogenated Coco-Glycerides, Butyrospermum Parkii Butter, Theobroma Cacao (Cocoa) Seed Butter, Mangobutter, Methyl Palmitat, Hydrogenated Palm Kernel Glycerides and Hydrogenated Palm Glycerides (Lipocire A, Gattefosse) , Acacia Decurrens/Jojoba/Sunflower Seed Wax Polyglyceryl-3 Esters, Cetearyl Nonanoate und/oder Cetearyl Alcohol.

6. Zubereitungen nach einem der vorstehenden Ansprüche, umfassend mehr als 0,5 Gew.-%, vorteilhaft 1 bis 10 Gew.-%, jeweils bezogen auf die Gesamtmasse der Dispersion, an einem oder mehreren Ölen.

## Claims

1. Cosmetic or dermatological preparations in the form of hydrodispersions,
a) consisting of a liquid lipid phase in an outer aqueous phase,
b) wherein a gel structure is formed in the aqueous phase with the aid of one or more gel formers and/or one or more thickeners, in which the lipid droplets are in stable suspension,
c) wherein the oil phase comprises one or more waxes,
d) wherein the oil phase has a content of 2-ethylhexyl salicylate and 4-tert-butyl-4'-methoxydibenzoylmethane and
e) the preparations are free from emulsifiers,
**characterized in that** they have the following compositions:
f) 60-95% by weight, in particular 70-85% by weight of water,
g) 0.05-5% by weight of at least one thickener,
h) up to 20% by weight, in particular more than 0.5% by weight, advantageously 1 to 10% by weight, of one or more oils, based in each case on the total mass of the dispersion, wherein the oil phase comprises 2 to 20% by weight, based on the total mass of the dispersion, of one or more waxes, wherein at least one wax has a melting point T_{onSet} < 30°C,
i) the thickener(s) is/are selected from the group of
• the organic natural compounds such as, in particular, agar-agar, carrageenan, tragacanth, gum arabic, alginates, pectins, polyoses, guar flour, carob seed flour, starch, dextrins, gelatins, casein, xanthan gum,
• the organic modified natural substances such as, in particular, carboxymethyl cellulose, hydroxyethyl and/or hydroxypropyl cellulose, microcrystalline cellulose.

2. Preparations according to the preceding claim, **characterized in that** sodium polyacrylate is selected as additional thickener.

3. Preparations according to either of the preceding claims, **characterized in that** the proportion of thickeners is selected in the range of 0.1 to 2% by weight, based on the total mass of the hydrodispersion preparation.

4. Preparations according to any of the preceding claims, **characterized in that** the wax(es) having a T_{onSet} below 30°C are selected from the group comprising fats (triglycerides), mono- and diglycerides, natural and synthetic waxes, fatty alcohols and wax alcohols, fatty acids, esters of fatty alcohols and fatty acids, and fatty acid amides or any desired mixtures of these substances.

5. Preparations according to any of the preceding claims, **characterized in that** the wax(es) is/are selected from the group comprising Shorea stenoptera seed butter, hydrogenated vegetable oil, hydrogenated coco-glycerides, Butyrospermum parkii butter, Theobroma cacao (cocoa) seed butter, mango butter, methyl palmitate, hydrogenated palm kernel glycerides and hydrogenated palm glycerides (Lipocire A, Gattefossé), Acacia decurrens/jojoba/sunflower seed wax polyceryl-3 esters, cetearyl nonanaote and/or cetearyl alcohol.

6. Preparations according to any of the preceding claims, comprising more than 0.5% by weight, advantageously 1 to 10% by weight, based in each case on the total mass of the dispersion, of one or more oils.

## Revendications

1. Préparations cosmétiques ou dermatologiques sous forme d'hydrodispersions,
a) qui sont constituées par une phase lipidique liquide dans une phase aqueuse externe,
b) une structure de gel étant construite dans la phase aqueuse à l'aide d'un ou de plusieurs gélifiants et/ou d'un ou de plusieurs épaississants, dans laquelle structure les gouttes lipidiques sont mises en suspension de manière stable,
c) la phase huileuse contenant une ou plusieurs cires,
d) la phase huileuse présentant une teneur en salicylate de 2-éthylhexyle et en 4-tert-butyl-4'-méthoxydibenzoylméthane et
e) les préparations étant exemptes d'émulsifiants,
**caractérisées en ce qu'**elles présentent les compositions suivantes :
f) 60-95% en poids, en particulier 70-85% en poids d'eau
g) 0,05-5% en poids d'au moins un épaississant,
h) jusqu'à 20% en poids d'une ou de plusieurs huiles, en particulier plus de 0,5% en poids, avantageusement 1 à 10% en poids, à chaque fois par rapport à la masse totale de la dispersion, la phase huileuse contenant 2 à 20% en poids, par rapport à la masse totale de la dispersion, d'une ou de plusieurs cires, au moins une cire présentant un point de fusion T_{onSet} < 30°C,
i) **en ce que** le ou les épaississants sont choisis dans le groupe
des composés organiques naturels, tels qu'en particulier l'agar-agar, le carrag-hénane, la gomme adragante, la gomme arabique, les alginates, les pectines, les polyoses, la farine de guar, la farine de graines de caroube, les amidons, les dextrines, les gélatines, la caséine, la gomme de xanthane,
des substances naturelles organiques, modifiées, telles qu'en particulier la carboxy-méthylcellulose, l'hydroxyéthylcellulose et/ou l'hydroxypropylcellulose, la cellulose microcristalline.

2. Préparations selon l'une quelconque des revendications précédentes, **caractérisées en ce que** le polyacrylate de sodium est choisi comme épaississant supplémentaire.

3. Préparations selon l'une quelconque des revendications précédentes, **caractérisées en ce que** la proportion d'épaississants est choisie dans la plage de 0,1 à 2% en poids, par rapport à la masse totale de la préparation d'hydrodispersion.

4. Préparations selon l'une quelconque des revendications précédentes, **caractérisées en ce que** la cire ou les cires présentant un T_{onSet} inférieur à 30°C est/sont choisie(s) dans le groupe constitué par les graisses (triglycérides), les monoglycérides et les diglycé-rides, les cires naturelles et synthétiques, les alcools de graisse et de cire, les acides gras, les esters d'alcools gras et d'acides gras ainsi que les amides d'acides gras ou les mélanges quelconques de ces substances.

5. Préparations selon l'une quelconque des revendications précédentes, **caractérisées en ce que** la cire ou les cires est/sont choisie(s) dans le groupe constitué par le beurre d'il-lipé, l'huile végétale hydrogénée, les glycérides hydrogénés de coco, le beurre de karité, le beurre de cacao, le beurre de mangue, le palmitate de méthyle, les glycérides hydrogénés de palmiste et les glycérides hydrogénés de palme (Lipocire A, Gattefosse), les esters de polyglycéryle-3 de cire de graines d'Acacia Decurrens/jojoba/tournesol, le nonanoate de cétéaryle et/ou l'alcool cétéarylique.

6. Préparations selon l'une quelconque des revendications précédentes, comprenant plus de 0,5% en poids, avantageusement 1 à 10% en poids, à chaque fois par rapport à la masse totale de la dispersion, d'une ou de plusieurs huiles.
